# EUROPEAN PATENT APPLICATION

(11) **EP 1 810 683 A1**
(43) Date of publication of application: **25.07.2007**
(21) Application number: 05726995.3
(22) Date of filing: 24.03.2005
(51) Int. Cl.: A61K 33/38, A61P 35/00, A61P 37/04, C02F 1/28, C02F 1/42, C02F 1/46, C25B 1/04, C25B 1/10, C25B 9/18, C25B 11/04

(54) **IMMUNOPOTENTIATOR AND METHOD AND APPARATUS FOR PRODUCING THE SAME**

(30) Priority: 27.09.2004 JP 2004279254
(71) Applicant: HOSHIZAKI DENKI KABUSHIKI KAISHA, Toyoake-shi, Aichi 470-1194 (JP)
(72) Inventor: INAMORI, Soichiro c/o HOSHIZAKI DENKI K. K., Toyoake-shi, Aichi 4701194 (JP); HAYASHI, Masahiko, Kawasaki-shi Kanagawa 2130034 (JP)
(74) Representative: Kramer - Barske - Schmidtchen
(86) International application number: PCT/JP2005/005423
(87) International publication number: WO 2006/035523

(57) **Abstract**

An immunopotentiator to be used for enhancing an immune function of suppressing the proliferation of allogeneic tumor, the immunopotentiator containing, as an active ingredient, electrolyzed weakly alkaline water produced by electrolysis of water containing a silver component

## Description

### Field of the Invention

The present invention relates to an immunopotentiator to be used for enhancing an immune function of suppressing the proliferation of allogeneic tumor cells, and a method and apparatus for producing the immunopotentiator.

### Discussion of the prior art

There have been developed various kinds of tumor treatments for curing malignant tumor such as cancer. These tumor treatments are in the form of a medicine containing, as an effective component, a chemical material which causes various side effects in a different phase. In recent years, there has been proposed in Japanese patent laid-open publication No. 2001-137852 a specific kind of tumor treatments in the form of a cancer suppressor containing, an effective component, electrolytic reduction water produced by electrolysis of NaOH water. There has been also proposed in a re-laid open patent publication WO95/14484 an in-vivo free-radical generator in the form of an organic or inorganic ion exchanger deposited with silver ion.

The cancer suppressor proposed in the former patent publication was developed on a basis of the fact that dissolved hydrogen of high concentration contained in electrolytic reduction water (electrolyzed alkaline water) is effective to prevent or repair damage of DNA caused by active oxygen. The electrolytic reduction water containing the dissolved hydrogen is, in general, produced by electrolysis of diluted water of NaCl in a cathode chamber of an electrolytic cell with a partition membrane.

In electrolysis of diluted water of NaCl in the electrolytic cell, electrolyzed acidic water containing chlorine is produced in an anode chamber of the electrolytic cell, and a large amount of hypochlorous acid and chlorine gas generated in the anode chamber dissolves in the electrolytic reduction water. Such electrolytic reduction water containing a large amount of chlorine component is unsuitable for dose, and the dissolved chlorine component is carcinogenic to tumor cell. For this reason, NaOH solution substituted for NaCl solution is used to produce electrolyzed reduction water without chlorine component.

The in-vivo free-radical generator proposed in the latter patent publication was developed on a basis of the fact that · OH of free-radical is effective to destruct tumor cell. In the development of the in-vivo free-radical generator, it has been found that radical decomposition of water is caused by silver ion to continually generate · OH for a long period of time. From this point of view, an ion exchanger deposited with silver ion is specified as the in-vivo free-radical generator.

### Summery of the Invention

As a result of vigorous research of specific function of electrolyzed water, the inventors have found the fact that electrolyzed alkaline or acidic water containing silver component does not effect to directly attack cancer cells but effective to enhance the immune function to a specific tumor. The present invention was made on a basis of the above fact. It is, therefore, an object of the present invention to provide an immunopotentiator containing, as an active ingredient, electrolyzed water containing a specific component, a method of producing the immunopotentiator and an apparatus for producing the same.

According to the present invention, the object is accomplished by providing an immunopotentiator to be used for enhancing an immune function of suppressing the proliferation of allogeneic tumor, the immunopotentiator containing, as an active ingredient, electrolyzed weakly alkaline water produced by electrolysis of water containing a silver component. In the immunopotentiator, it is preferable that the electrolyzed weakly alkaline water is prepared at pH 8.0 ~ 10.0 and contains the silver component of 30 ppb ~ 200 ppb. In the immunopotentiator, it is also preferable that the electrolyzed weakly alkaline water is prepared at an oxide reduction potential of +400 mV ~ -800 mV.

The object of the present invention is also accomplished by providing an immunopotentiator to be used for enhancing an immune function of suppressing the proliferation of allogeneic tumor, the immunopotentiator containing, as an active ingredient, electrolyzed weakly acidic water produced by electrolysis of water containing a silver component. In the immunopotentiator, it is preferable that the electrolyzed weakly acidic water is prepared at pH 8.0 ~ 10.0 and contains the silver component of 30 ppb ~ 200 ppb. In the immunopotentiator, it is also preferable that the electrolyzed weakly acidic water is prepared at an oxide reduction potential of + 200 mV ~ +800 mV

Although the immunopotentiator does not effect as a carcinostatic to directly attack a tumor such as carcinomas, it is effective to suppress the proliferation of mouse tumor such as Sarcoma 180 (S-180) but ineffective to suppress the proliferation of mouse tumor such as Meth-A. Such effect as anti-tumor function is presumed in immunity by the fact that S-180 is an allogeneic tumor of high antigenicity while Meth-A is a syngeneic tumor. As a result of inspection of activated reaction of lienorenal lymphocyte based on the above fact, it is has been confirmed that the manifestation rate of cell surface marker CD 233 indicative of activation of natural killer and the manifestation rate of CD3e indicative of cytopathy and T-cell activation noticeably increase in tumor suppression groups. These results suggest the fact that cellular immune function in tumor suppression groups are activated by dose of electrolyzed alkaline or acidic water containing a silver component specified by the present invention.

From the foregoing facts, it has been recognized that the electrolyzed alkaline or acidic water containing a silver component specified by the present invention is effective to enhance anti-tumor immune reaction to allogeneic tumor cells and acts as an immunopotentiator for enhancing an immune function.

The immunopotentiator according to the present invention can be produced by electrolysis of water containing the silver component in a cathode chamber of an electrolytic cell with a partition membrane or without any partition membrane. The immunopotentiator may be produced by electrolysis of water containing the silver component in an anode chamber of an electrolytic cell with a partition membrane or without any partition membrane.

Furthermore, the immunopotentiator may be produced by electrolysis of solution of silver powder solved in pure water in a cathode chamber of an electrolytic cell with a partition membrane or without any partition membrane.

According to the present invent ion, there is provided a production apparatus of the immunopotentiator, which comprises a water treatment system composed of a water softener for softening general water such as tap water or natural water and a water purifier for purifying the softened water, a first electrolytic cell provided at its anode side with a silver electrode for electrolyzing the purified water, and a second electrolytic cell with a partition membrane for electrolyzing the electrolyzed water produced in the first electrolytic cell.

According to an aspect of the present invention, there is provided a production apparatus of the immunopotentiator, which comprises a water treatment system composed of a water softener for softening general water such as tap water or natural water and a water purifier for purifying the softened water, a first electrolytic cell provided at its anode side with a silver electrode for electrolyzing the purified water, and a second electrolytic cell for electrolyzing the electrolyzed water produced in the first electrolytic cell, the second electrolytic cell having anode and cathode chambers in connection with discharge conduits for discharging the electrolyzed water therefrom.

### Brief description of the drawings

In the drawings:
Fig. 1 is a schematic illustration of a production apparatus of an immunopotentiator according to the present invention;
Fig. 2 is a graph showing a change of proliferation (estimated weight) of grafted S-180 tumor in lapse of time;
Fig. 3 is a graph showing the proliferation (real weight) of grafted S-180 tumor,
Fig. 4 is a graph showing a change of proliferation (estimated weight) of grafted Meth-A tumor in lapse of time;
Fig. 5 is a graph showing the proliferation (real weight) of grafted Meth-A, tumor,
Fig. 6 is a graph showing a suppressive effect in lapse of time of each reagent (distilled water, PSK, Ag0) to the proliferation of grafted S-180 tumor;
Fig. 7 is a graph showing a suppressive effect in lapse of time of each reagent (distilled water, Ag40) to the proliferation of grafted S-180 tumor;
Fig. 8 is a graph showing a graph showing a suppressive effect in lapse of time of each reagent (distilled water, Ag70) to the proliferation of grafted S-180 tumor,
Fig. 9 is a graph showing a suppressive effect in lapse of time of each reagent (distilled water, Ag150) to the proliferation of grafted S-180 tumor;

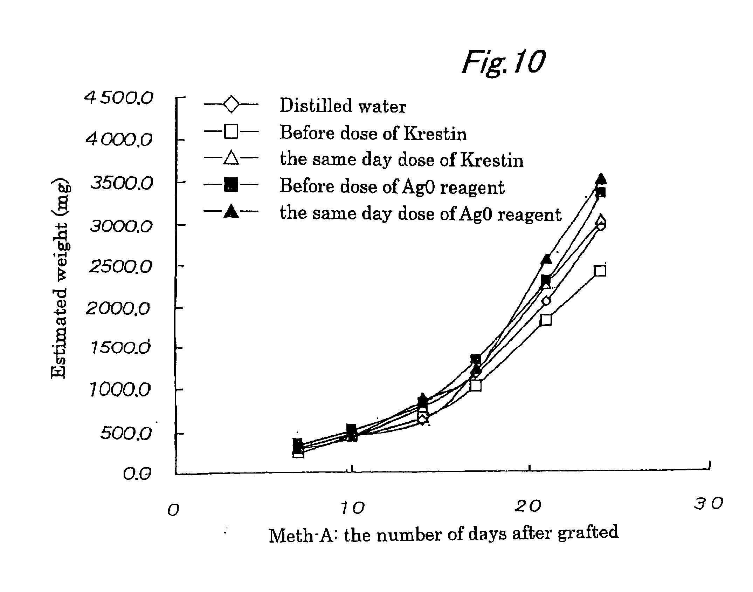

Fig. 10 is a graph showing a suppressive effect in lapse of time of each reagent (distilled water, PSK, Ag0) to the proliferation of grafted Meth-A tumor;

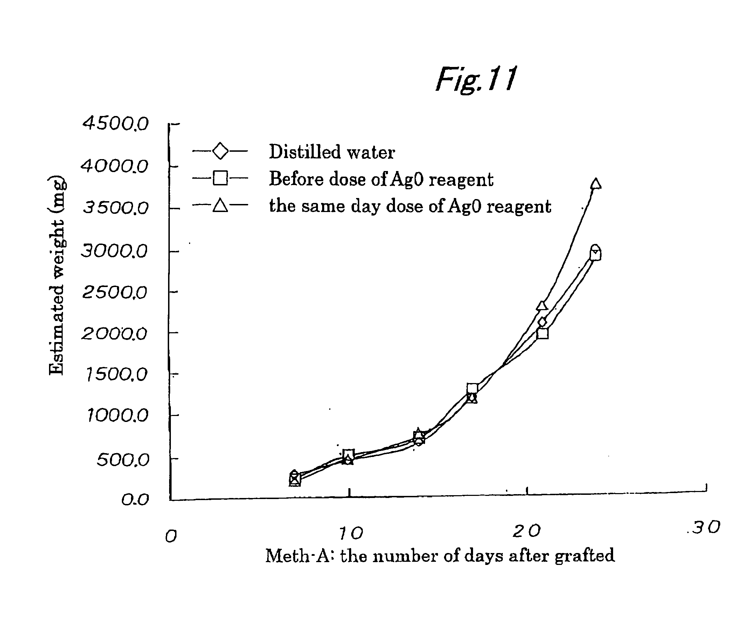

Fig. 11 is a graph showing a suppressive effect in lapse of time of each reagent (distilled water, Ag40) to the proliferation of grafted Meth-A tumor;

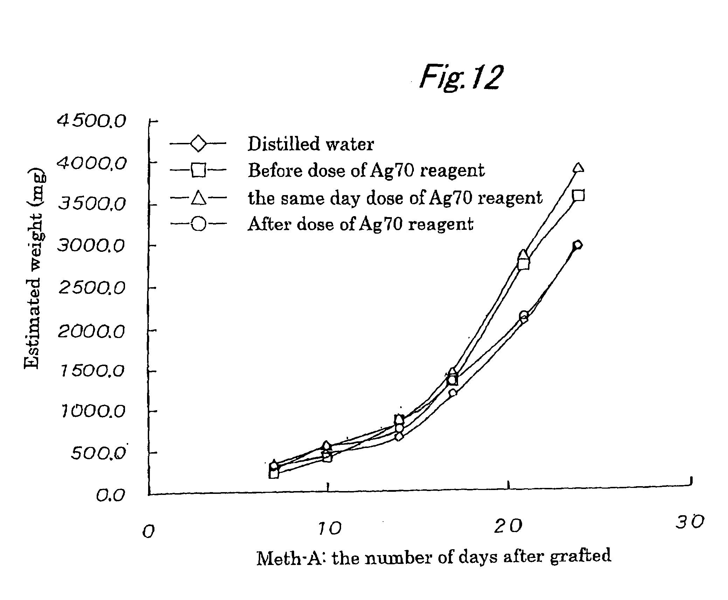

Fig. 12 is a graph showing a suppressive effect in lapse of time of each reagent (distilled water, Ag70) to the proliferation of grafted Meth-A tumor;

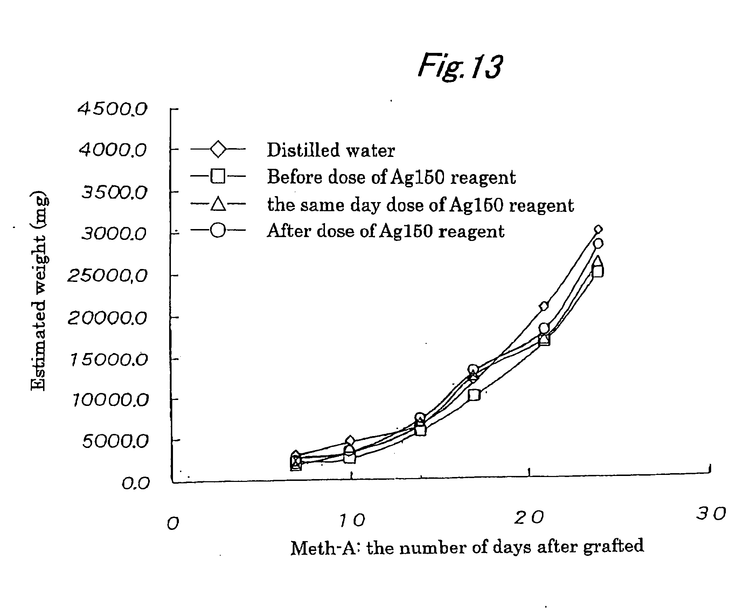

Fig. 13 is a graph showing a suppressive effect in lapse of time of each reagent (distilled water, Ag150) to the proliferation of grafted Meth-A tumor;
Fig. 14 illustrates an analyzed result of each group of tumor dosed with reagents;
Fig. 15 is a graph showing a suppressive effect (7^{th} day) of each reagent to the proliferation of grafted S-180 tumor;
Fig. 16 is a graph showing a suppressive effect (10^{th} day) of each reagent to the proliferation of grafted S-180 tumor;
Fig. 17 is a graph showing a suppressive effect (14^{th} day) of each reagent to the proliferation of grafted S-180 tumor;
Fig. 18 is a graph showing a suppressive effect of each reagent in difference of production method to the proliferation of grafted 5-180 tumor;
Fig. 19 is a graph showing a suppressive effect of each reagent in difference of dose time to the proliferation of grafted S-180 tumor; and
Fig. 20 is a graph showing a suppressive effect of each reagent in difference of the concentration of silver component to the proliferation of grafted S-180 tumor.

### Preferred embodiment of the Invention:

The present invention relates to an immunopotentiator, and a method and apparatus for producing the immunopotentiator. The immunopotentiator according to the present invention is provided to enhance the immune function suppressing the proliferation of allogeneic tumor cell. A first immunopotentiator contains, as an effective component, electrolyzed alkaline water containing a silver component. In the first immunopotentiator, the pH of the electrolyzed alkaline water is in an extent of pH 8.0 ~ 10.0, the oxide reduction potential is in a range of +400 mV ~ +800 mV, and the silver component is in an extent of 30 ppb ~ 200 ppb in terms of silver ion.

A second immunopotentiator contains, as an effective component, electrolyzed acidic water containing a silver component In the second immunopotentiator, the pH of electrolyzed acidic water is in an extent of pH5.0 ~ 7.0, the oxide reduction potential is in an extent of +200 mV ~ + 800 mV, and the silver component is in an extent of 30 ppb ~ 200 ppb in terms of silver ion. Although these immunopotentiators do not effect as a carcinostatic to directly attack a tumor such as carcinomas, it has been confirmed that the immunopotentiators are effective to suppress the proliferation of mouse tumor such as Sarcoma 180 (S-180) but ineffective to suppress the proliferation of mouse tumor such as Meth-A. Such effect as anti-tumor function are suggested in immunity by the fact that Sarcoma 180 (S-180) is an allogeneic tumor of high antigenicity while Meth-A is a syngeneic tumor.

As a result of inspection of activated reaction of liennorenal lymphocyte based on the above fact, it has been confirmed that the manifestation rate of cell surface marker CD 233 indicative of natural killer and the manifestation rate of CD3e indicative of cytopathy and T-cell activation are noticeably increased in tumor suppression groups. These results suggest the fact that cellular immune function in tumor suppression groups are activated by dose of electrolyzed alkaline or acidic water containing a silver component specified by the present invention

Although the function and immunological mechanism of the electrolyzed alkaline or acidic water containing the silver component are still indefinite, it has been found that the electrolyzed alkaline or acidic water is useful as an immunopotentiator for enhancing anti-tumor immune function against an allogeneic tumor of high antigenicity and acquired immunity. The immunopotentiation is enhanced in accordance with an amount of silver component In the case that the containing amount of silver component is 150 ppb, it has been confirmed that the immunopotentiation is maximized.

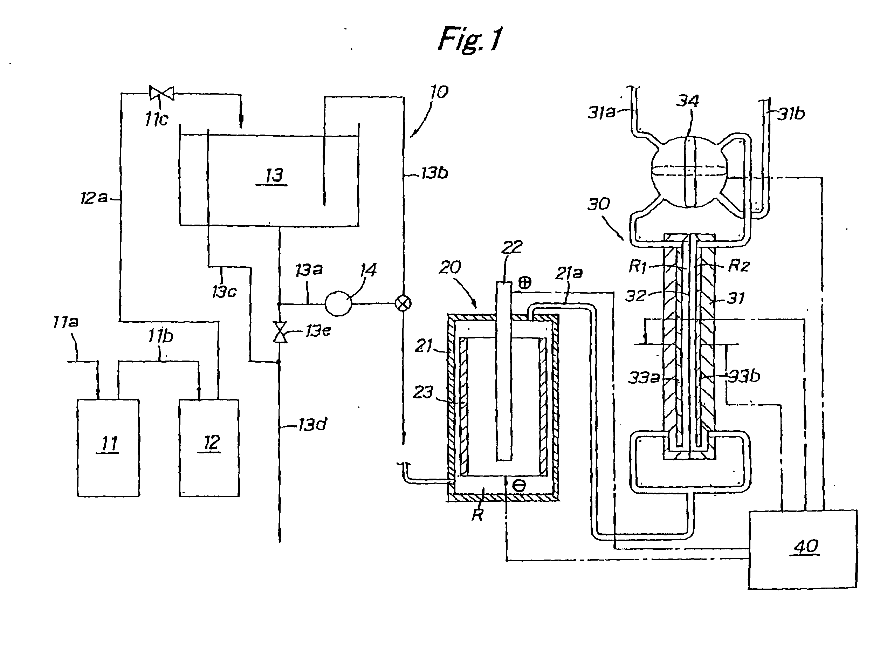

The immunopotentiator can be produced by electrolysis of softened and purified tap-water or natural water in an electrolytic cell without any partition membrane and electrolysis of electrolyzed water in an electrolytic cell with a partition membrane. In Fig. 1, there is schematically illustrated a production apparatus of the immunopotentiator which comprises a water treatment system 10, an electrolytic cell 20 without any partition membrane and an electrolytic cell with a partition membrane.

The water treatment system 10 includes a water softener 11, a water purifier 12 and a water storage tank 13. The water softener 11 is provided therein with an ion-exchange resin, and the water purifier 12 is provided therein with a filter element of high filtering function. The water softener 11 is connected to a source of tap-water through a water supply conduit 11a and connected to the water purifier 12 through a water supply conduit 11b. The water purifier 12 is connected to the water storage tank 13 through a water supply conduit 12a, and the water storage tank 13 is connected to an electrode chamber R of the electrolytic cell 20 through a water supply conduit 13a. The water storage tank 13 is provided with a circulation conduit 13b, an overflow conduit 13c and a water level sensor (not shown). The overflow conduit 13c is connected to a drain conduit 13d, the water supply conduit 13a is connected at its intermediate portion to the drain conduit 13d through a drain valve 13, and the water supply conduit 13a is provided with a water pump 14.

The water treatment system 10 is provided to store an amount of tap-water treated by water softener 11 and water purifier 12 in the water storage tank 13 and to supply the treated water from the water storage tank 13 to an electrode chamber R of the electrolytic cell 20. In the water treatment system 10, the water pump 14 is operated to supply the treated water into the electrode chamber R of electrolytic cell 20.

The electrolytic cell 20 has a cylindrical housing 21 which is provided therein with an anode electrode 22 and a cathode electrode 23. The anode electrode 22 is in the form of a rod of silver located at the center of cylindrical housing 21, and the cathode electrode 23 is in the form of a cylindrical body of SU304 arranged in a surrounding relationship with the anode electrode 22. The electrode chamber R formed in cylindrical housing 21 is connected at its bottom portion to the water storage tank 13 through the water supply conduit 13a and is connected at its upper portion to a discharge conduit 21a which is bifurcated for connection a pair of electrode chambers R1 and R2 of the electrolytic cell 30.

In the electrolytic cell 20, the anode and cathode electrodes 22 and 23 are applied with an electric voltage from a power source under control of an electric controller 40. During activation of the electrolytic cell 20, the water pump 14 is driven to supply the treated tap-water from the water storage tank 13 to the electrode chamber R. The treated tap-water is electrolyzed by an electrolytic current in the electrode chamber R, and the electrolyzed water is supplied to the electrode chambers of electrolytic cell 30. The value of electrolytic current is controlled by a microcomputer of the electric controller 40. During activation of the electrolytic cell 20, the value of electrolytic current between anode and box-type housing 31 the interior of which is subdivided into a pair of electrode cathode electrodes 22 and 23 is controlled in an extent of 0 ~ 30 mA.

The electrolytic cell 30 with the partition membrane is composed of a chambers R1. and R2 by means of an ion-permeable partition membrane 32. Electrodes 33a and 33b are disposed in the electrode chambers R1 and R2 through the partition membrane 32. The electrodes 33a, 33b each are in the form of a titanium plate plated with Pt and opposed to each other through the partition member 32. The electrolytic cell 30 is connected at its bottom portion to a discharge conduit 21 a of electrolytic cell 20 to be supplied with electrolyzed water from the electrolytic cell 20. A pair of discharge conduits 31a and 31b are connected to the electrode chambers R1 and R2 of housing 31, and a changeover valve 34 is disposed in the discharge conduits 31a and 31b.

The electrodes 33a and 33b of electrolytic cell 30 are applied with an electric voltage from the power source under control of the controller 40 during activation of the electrolytic cell 20. During activation of the electrolytic cell 30, the polarity of the electrodes is reversed upon each lapse of a predetermined time, and the changeover valve 34 is operated in response to the reverse of polarity to switchover the connection of discharge conduits 31a, 31b to the electrode chambers R1, R2. The reverse of polarity and the operation of changeover valve 34 are controlled by the microcomputer of controller 40. During activation of the electrolytic cell 30, the electric current between the electrodes 33a and 33b is maintained in an extent of 0 ~ 3A.

The electrode chambers R1 and R2 are periodically reversed from an anode chamber to a cathode chamber or vice versa respectively in response to the reverse of polarity to remove scale adhered to the electrodes. In a condition where the electrode chamber R1 is an anode chamber while the electrode chamber R2 is a cathode chamber, electrolyzed acidic water is produced in the electrode chamber R1 while electrolyzed alkaline water is produced in the electrode chamber R2. As the changeover valve 34 is operated in response to the reverse of polarity, the electrolyzed acidic water is discharged through one of the discharge conduits 31a, 31b while the electrolyzed alkaline water is discharged through the other discharge conduit. In this embodiment, the electrolyzed alkaline water (containing a sliver component) is used as a first immunopotentiator, and the electrolyzed acidic water (containing a silver component) is used as a second immunopotentiator.

In the production system of immunopotentiator, the water treatment system 10 is activated to store a predetermined amount of treatment water in the water storage tank 13, and the water pump 14 is operated to supply the treated water into the electrode chamber R of the electrolytic cell 20. When the treated water in the water storage tank13 decreases less than a predetermined level, the microcomputer of controller 40 outputs a detection signal detected by the water level sensor, and a water supply valve 11 c is opened in response to the detection signal applied from the microcomputer to supply the treated water into the water storage tank 13.

During synchronous activation of the electrolytic cells 20 and 30, the electrodes 22 and 23 of electrolytic cell 20 are applied with electrolysis current less than 30 mA, and the electrodes 33a and 33b of electrolytic cell 30 are applied with electrolysis current less than 3A. In the electrolytic cell 20, a silver ion from the anode electrode 22 dissolves in the treatment water supplied to the electrolysis chamber R. Thus, electrolyzed neutral water containing the silver ion is produced in the electrolytic cell 20 and supplied to the electrolysis chambers R1 and R2 of electrolytic cell 30.

In a condition where the electrolysis chamber R1 of electrolytic cell 30 is an anode chamber while the electrolysis chamber R2 is a cathode chamber, electrolyzed acidic water is produced in the electrolysis chamber R1 while electrolyzed alkaline water is produced in the electrolysis chamber R2. In a condition where the electrolysis chamber R1 of electrolytic cell 30 is a cathode chamber while the electrolysis chamber R2 is an anode chamber, electrolyzed alkaline water is produced in the electrolysis chamber R1 while electrolyzed acidic water is produced in the electrolysis chamber R2. The electrolyzed acidic water is discharged into a predetermined place through the discharge conduit 31a, and the electrolyzed alkaline water is discharged into another place through the discharge conduit 31b. Thus, the electrolyzed alkaline water containing an amount of silver component is used as the first immunopotentiator, and the electrolyzed acidic water containing an amount of silver component is used as the second immunopotentiator.

In the production system of immunopotentiator, the amount of silver component contained in the electrolyzed water can be adjusted by an operative condition of the electrolytic cell 20. For instance, the concentration of silver ion can be adjusted in an extent of 0 ~ 200 ppb under control of the electrolysis current and time. The characteristic of immunopotentiator such as pH can be controlled by an operative condition of the electrolytic cell 30. For instance, the pH of the electrolyzed alkaline water can be adjusted in an extent of 7.0 ~ 11.0 under control of the electrolysis current and time, the pH of the electrolyzed acidic water can be adjusted in an extent of 3.0 ~ 7,0.

### Embodiment 1:

With respect to electrolyzed alkaline water of different concentration produced in the production apparatus of immunopotentiator, the following two experiments (in vitro) were carried out to confirm the suppressive function of proliferation of tumor cells and the anti-tumor function to grafted tumor.

### 1) Experiment 1: Function to various tumor cells

In this experiment, cancer cells of various mouse and human being cancer cells preserved for passage in the laboratory of Institute of Kitasato were adapted as test samples. Four kinds of electrolyzed alkaline water produced in the production apparatus of the immunopotentiator were used as reagents 1 ~ 4. The reagent 1 is electrolyzed alkaline water of pH 9.3 and containing a silver component of 50 ppb, the reagent 2 is electrolyzed alkaline water of pH 9.3 and containing a silver component of 25 ppb, the reagent 3 is electrolyzed alkaline water of pH 9.3 and containing a silver component of 12.5 ppb, and the reagent 4 is electrolyzed alkaline water of pH 9.3 and containing a silver component of 6.25 ppb. In the test samples, mouse melanoma cell strain (B16/BL6), sarcoma cell strain (S-180), mouse lymphoma cell strain (P388), fibro-sarcoma cell strain (L929), human neuroblastoma cell strain (NB-1), human uter-cervical cancer cell strain (HeLa), and human fibroblast cell strain (WI-38 cell) were preserved for passage on Minimum Eagle's Medium (MEM) containing 10 % fetal bovine serum (FBS). These cells were prepared in 3 ~ 5 x 10⁴ cell /mL, and the prepared cells of 0.1 mL were disseminated on a culture plate of 96 holes. The cells were added with a target amount of the reagents and further added with MEM medium to be 0.2 mL in total. Thus, the cells were cultured for 72 hours at 37 °C under the presence of 5 % CO₂. The survival rate of the cells were measured by colorimetry of MTT Method (Carmichaei, J., et al. Cancer Res. Vol. 47, p936 ~ 942, 1987).

In the test samples, mouse sarcoma cell strain (Meth - A), mouse lung Cancer strain (LCC), human stomach cancer strain (KATO-III), human myelogenic leukemia cell strain (HL-60), human histiocytic lymphocytoma strain (U-937), human prostate cancer strain (LNCAP), mouse lymphocytoma strain (L5178Y-ML), and human lymphoblastic leukemia cell strain (Jurkat) were preserved for passage on a culture medium containing 10 % FBS. These cells were prepared in 5 x 10⁴ cell/mL, and the prepared cel ls of 0. 1 mL were disseminated on a culture plate of 96 holes. The cells were added with a target amount of the reagents and further added with culture medium (RPMI-1640) to be 0.2 mL in total. Thus, the cells were cultured for 72 hours at 37 °C under the presence of 5 % CO₂. The survival rate of the cells were measured by colorimetry of MTT method.

In the test samples, mouse myeloblastic leukemia cell strain (M1), human liver cancer strain (Hep G2), and human cavum-oris epithelial cancer strain (KB cell) were preserved for passage on DMEM (Dulbeccs Moijed EM) medium containing 10 % FBS. These cells were prepared in 5 x 10⁴ cell/mL, and the prepared cells of 0.1 mL were disseminated on the culture plate of 96 holes. The cells were added with a target amount of the reagents and further added with DMEM medium to be 0.2 mL in total. Thus, the cells were cultured for 72 hours at 37 °C under the presence of 5 % CO₂. The survival rate of the cells were measured by colorimetry of MTT method. A result of the experiments is shown in the folloing table 1 (Survival rate of mouse established cancer cells) and table 2 (Survival rate of human established cancer cells)

**Table 1**

| Test samples | Survival rate % (colorimetry of MTT method) | | | |
|---|---|---|---|---|
| | Reagent 1 | Reagent 2 | Reagent 3 | Reagent 4 |
| mouse melanoma cell strain (B 16BL6) | 103.6 | 100.2 | 99.7 | 109.0 |
| sarcoma cell strain (S-180) | 94.5 | 100.6 | 105.6 | 100.8 |
| mouse sarcoma cell strain (Meth-A) | 99.7 | 96.7 | 104.3 | 105.1 |
| mouse myeloblastic leukemia cell strain (M1) | 102.3 | 98.8 | 104.3 | 98.2 |
| mouse lymphocytoma strain (L5178Y-ML) | 93.5 | 97.6 | 99.9 | 99.5 |
| mouse lymphoma cell strain (P388) | 90.4 | 98.8 | 99.7 | 100.1 |
| fibrco-sarcoma cell strain (L929) | 100.5 | 108.7 | 106.6 | 98.9 |
| mouse lung cancer strain (LLC) | 106.6 | 110.6 | 110.2 | 108.6 |

| | | | | |
|---|---|---|---|---|
| Reagent 1: Electrolyzed alkaline water of pH 9.3 containing a sliver component of 50 ppb Reagent 2: Electrolyzed alkaline water ofpH 9.3 containing a sliver component of 25 ppb Reagent 1: Electrolyzed alkaline water of pH 9.3 containing a sliver component of 12.5 ppb Reagent 1: Electrolyzed alkaline water of pH 9.3 containing a sliver component of 6.25 ppb | | | | |

**Table 2**

| Survival rate of human established cancer cells | | | | |
|---|---|---|---|---|
| Test samples | Survival rate % (colorimetry of MTT method) | | | |
| | Reagent 1 | Reagent 2 | Reagent 3 | Reagent 4 |
| human stomach cancer strain (KATO-III) | 103.3 | 108.5 | 105.9 | 108.5 |
| human liver cancer strain (Hep G2) | 100.2 | 98.4 | 102.5 | 110.6 |
| human myelogenic leukemia cell strain (HL-60) | 100.1 | 100.5 | 108.8 | 111.2 |
| human histiocytic lymphocytoma strain (U-937) | 103.3 | 97.6 | 98.8 | 113.4 |
| Human neuroblastoma cell strain (NB-1) | 101.5 | 110.2 | 111.8 | 106.7 |
| Human uter-cervical cancer cell strain(HeLa) | 98.6 | 111.2 | 106.7 | 103.3 |
| human prostate cancer strain (LNCAP) | 109.8 | 98.8 | 103.5 | 113.5 |
| human fibroblast cell strain (W1-38 cell) | 108.8 | 111.5 | 110.8 | 99.4 |
| Human lymphoblastic leukemia cell strain (Jurkat) | 100.1 | 98.8 | 99.6 | 106.4 |
| human cavum-oris epithelial cancer strain (KB) | 106.6 | 101.1 | 100 | 104.1 |

| | | | | |
|---|---|---|---|---|
| Reagent 1: Electrolyzed alkaline water of pH 9.3 containing a sliver component of 50 ppb Reagent 2: Electrolyzed alkaline water of pH 9.3 containing a sliver component of 25 ppb Reagent 1: Electrolyzed alkaline water of pH 9.3 containing a sliver component of 12.5 ppb Reagent 1: Electrolyzed alkaline water of pH. 9.3 containing a sliver component of 6.25 ppb | | | | |

### 2) Experiment 2: [Anti-tumor function to mouse]

In this experiment, 5-week age female mouse of ddY type (obtained from Japan SLC) and 5-week age female mouse of BALB/c-type (obtained from Japan SLC) were adapted as sample animals. The mouse of ddY type was grafted with Meth-A cell in its abdominal skin and dosed with reagents by free ingestion for fifteen days from the grafted day. For this period of time, the size (long and short radiuses) of tumor was periodically measured from the exterior, and the weight of the mouse was measured. After fifteen days, the mouse was killed by exsanguination, and the weight of tumor extracted from the mouse was measured.

In the experiment, electrolyzed alkaline water of pH 9.2 containing silver component of 70 ppb, and distilled water was substituted for the reagents as a control group. As comparative group, Krestin (Registered Tradmark) (PSK, positive control: Sankyo Kabushiki Kaisha) was substituted for the reagents. With respect to the animal groups, the mouse was provided for the experiment after preliminarily fed. For feeding, five mice were entered into a cage (32 x 231 x 13 cm) of polycarbonate and fed at a room temperature of 23 ± 0.5 °C under an ambience of 55 ± 5 % humidity. The cage was lightened for twelve hours from 8 am to 8 pm and conditioned for free ingestion of water and feed.

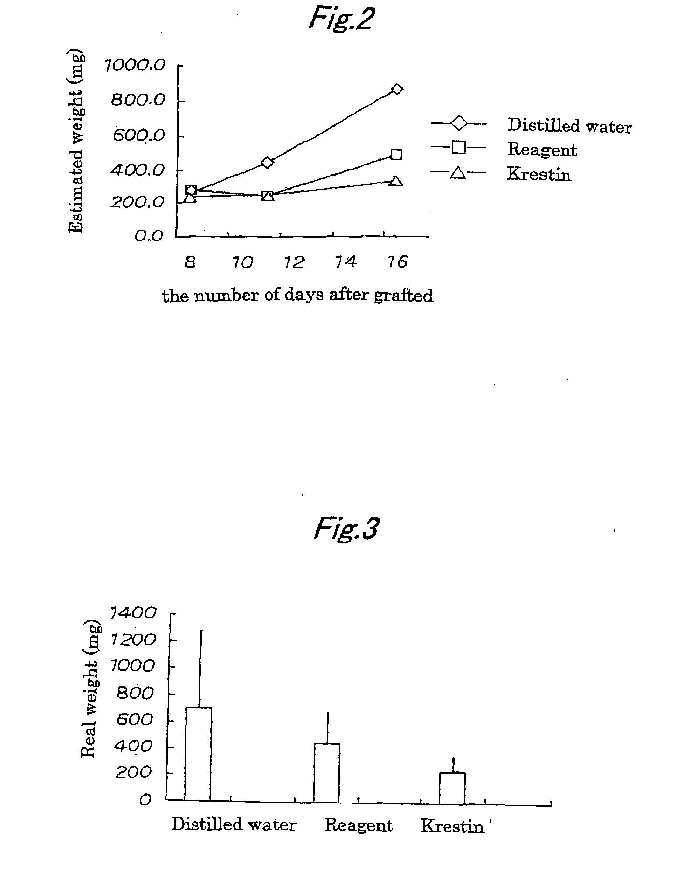

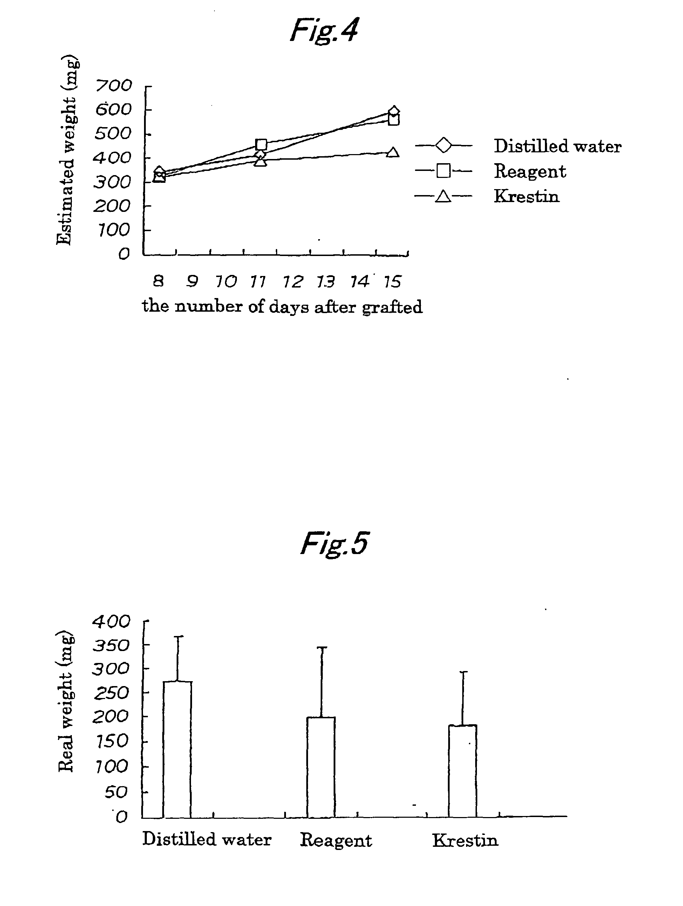

To confirm the proliferation degree of tumor, the size (long and short radiuses) of tumor was periodically measured by a slide calipers, and the estimated weight of tumor was calculated by a formula, the long radius (mm) x the short radius (mm)²/2. The statistic processing was made by an upaired t-inspection. The size of tumor extracted after dose of reagents was measured by direct measurement of the weight of extracted tumor. The suppressive effect (estimated weight and real weight) of proliferation of grafted S-180 tumor is shown in the following table 3, the variation in lapse of time of proliferation of the grafted S-180 tumor is shown in Fig. 2, and the real weight of proliferation of the grafted S-180 tumor is shown in Fig. 3. The suppressive effect (estimated weight and real weight) of proliferation of grafted Meth-A tumor is shown in the following table 4, the variation in lapse of time of proliferation of the grafted Meth-A tumor is shown in Fig.4, and the real weight of proliferation of the grafted Meth-A tumor is shown in Fig. 5.

**Table 3**

| Suppressive effect of proliferation of grafted S-180 tumor | | | | |
|---|---|---|---|---|
| Dosed reagent | Estimated weight | | | Real weight |
| | 8th Day | 11th Day | 16th Day | 16th Day |
| Distilled water | 254.7±11.2 | 446.1±112.5 | 866.1±481.8 | 701±570 |
| Reagent | 276.9±89.8 | 247.7±112.6 | 489.9±346.7 | 447±228 |
| Krestin | 234.0±101.5 | 246.4±108.1 | 337.7±201.2 | 238±113 |

| | | | | |
|---|---|---|---|---|
| Reagent: electrolyzed alkaline water of pH 9.2 containing silver component of 70 ppb Distilled water: control Krestin (PSK): positive control Weight: (Average value ± Standard deviation) mg (n = 7) | | | | |

**Table 4**

| Suppressive effect of proliferation of grafted Meth-A tumor | | | | |
|---|---|---|---|---|
| Dosed reagent | Estimated weight | | | Real weight |
| | 8th Day | 16th Day | 8th Day | Day 16 |
| Distilled water | 342.2±72.2 | 415.1±118.3 | 600.1±206.2 | 274±94 |
| Rreagent | 324.1±86.0 | 461.3±116.0 | 565.1±277.2 | 198±147 |
| Krestin | 317±95.8 | 389.8±134.3 | 428.8±260.9 | 138±110 |

| | | | | |
|---|---|---|---|---|
| Reagent: electrolyzed alkaline water of pH 9.2 containing silver component of 70 ppb Distilled water: control Krestin (PSK): positive control Weight: (Average value ± Standard deviation) mg (n = 7) | | | | |

### 3) Evaluation

In the inspection result of the function of the electrolyzed alkaline water containing a silver component to the proliferation of various kinds of mouse and human established cancer cell strains, it has been found that even if the concentration of the reagent in the mouse lymphocytoma strain (P388) high sensitive to suppression of the proliferation was a threshold value of 50 %, the suppression of proliferation more than about 10 % would not be effected. Similarly, the suppressive effect of proliferation was not found in the other cell strains. These results suggest the fact that the electrolyzed alkaline water containing a silver component does not effect to direct cytopathy, disconnection ofDNA/RNA, inhibition of transcription, inhibition of selective information transmission, and the like.

In the inspection result of the function of the electrolyzed alkaline water containing the silver component to the anti-tumor of the cells grafted in the mouse, it has been confirmed that the proliferation of S-180 cell grafted in skin is significantly suppressed in the same degree (P > 0.05) as in dose of the positive control (PSK). In the Meth-A cell grafted in skin, however, the proliferation of the cell was not suppressed since the S-180 cell is an alloantigen cell of high antigenicity while the Meth-A cell is an inbred cancer of low antigenicity. It is, in general, noted that PSK is an immunoreactive carcinostatic, while the suppressive function of proliferation of Meth-A is low. As the electrolyzed alkaline water containing the silver component has a suppressive function of the proliferation similar to the pattern of PSK, it is suggested that the suppressive function of the proliferation of the electrolyzed alkaline water is a function caused by immunity.

### Embodiment 2:

With respect to electrolyzed alkaline water of different concentration produced in the production apparatus of immunopotentiator, the following two experiments (in vitro) were carried to confirm the anti-tumor function to grafted mouse tumors (S-180, Meth-A). In this embodiment, S-180 tumor and Meth-A tumor preserved for passage in the laboratory of Institute of Kitasato were adapted as test samples. In this experiment, the electrolyzed alkaline water of different concentration was used as reagents 1 ∼ 4, distilled water was used as control, and Krestin, PSK of Sankyou Kabushiki Kaisha was used as positive control. The reagent 1 (Ag0) is electrolyzed alkaline water of pH 9.5 containing the silver ion of 0 ppb, the reagent 2 (Ag40) is electrolyzed alkaline water of pH 9.5 containing the silver ion 40 ppb, the reagent 3 (Ag70) is electrolyzed alkaline water of pH 9.5 containing the silver ion of 70 ppb, and the reagent 4w (Ag150) is electrolyzed alkaline water of pH 9.5 containing the silver ion 150 ppb. The reagents were produced everyday. A group of six mouses were used for each dose of the reagents.

### 1) Experiment 1: [Function to S-180]

In this experiment, 5-week age female mice of ddY type (obtained from Japan SLC) were used as sample mice. The mice of ddY type were grafted with S-180 cells of 0.5 x 10⁶ in its abodominal skin and dosed with the reagents , distilled water and PSK by free ingestion for twenty days from the grafted day. The mice were dosed with PSK by forced ingestion by using a probe. The ingestion dose was made on the same day from the grafted day to 24^{th} day (the same day dose), in one week from the grafted day to 24^{th} day (pre-dose), and in one week after the grafted day to 24^{th} day (after dose)

During the ingestion dose, the size (long and short radiuses) of tumor was periodically measured from the exterior, and the weight of the mice was measured After twenty days, the mice were killed by exsanguinations, and the weight of tumor extracted from the mice was measured. The mice were provided for the experiment after preliminarily fed For feeding, six mice were entered into a cage (32 x 231 x 13 cm) of polycarbonate and fed at a room temperature 23 ± 0.5°C under an ambience of 55 ± 5 % humidity. The cage was lightened for twelve hours from 8 a.m. to 8 p.m. and conditioned for free ingestion

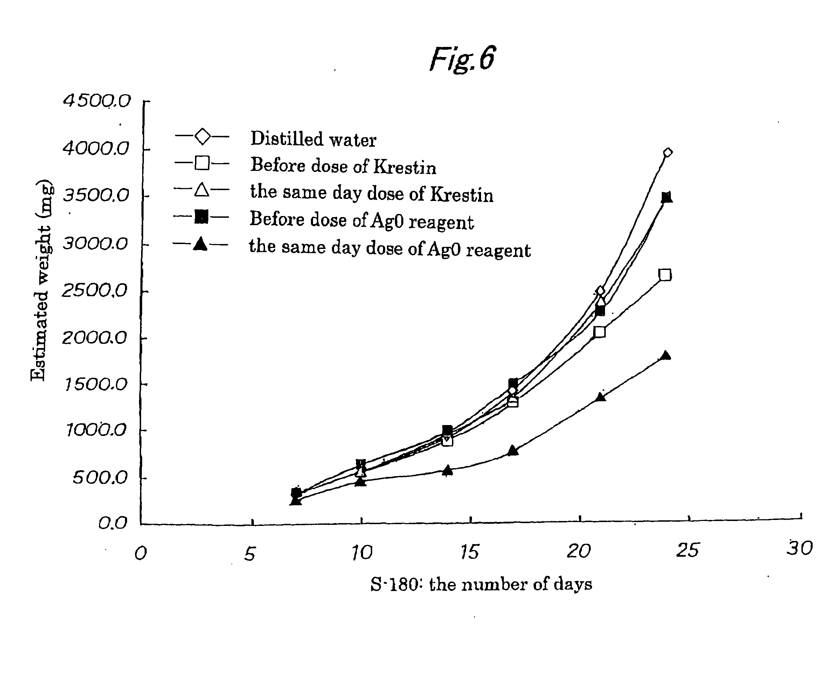

To confirm the proliferation degree of tumor, the size (long and short radiuses) of tumor was periodically measured by a slide calipers, and the estimated weight of tumor was calculated by a formula, the long radius (mm) x the short radius (mm)²/2. The statistic processing was made by an unpaired t-inspection. The size of tumor extracted after dose of the reagents was measured by direct measurement of the weight of extracted tumor. The suppressive effect (estimated weight and real weight) of proliferation of grafted S-180 tumor is shown in the following table 5, and the variation in lapse of time of proliferation of the grafted S-180 tumor is shown Figs. 6 ~ 9.

**Table 5**

| Suppressive effect of proliferation of grafted S-180 tumor | | | | | | |
|---|---|---|---|---|---|---|
| Dosed reagent | Estimated weight | | | | | |
| | 7 | 10 | 14 | 17 | 21 | 24 |
| Distilled water | 355.9 ±77.2 | 557.8 ±103.1 | 919.6 ±307.6 | 1420.5 ±445.9 | 2499.2 ±993.9 | 3974.9 ±1348.2 |
| Krestin (Pre-dose) | 321.2 ±59.3 | 550.4 ±112.2 | 877.3 ±341.6 | 1286.9 ±797.2 | 2052.7 ± 1339.8 | 2652.5 ± 1631.9 |
| Krestin (Same day dose) | 316.6 ±97.7 | 53.5 ±225.5 | 957.5 ±433.7 | 1345.6 ±756.3 | 2376.5 ±1377.5 | 3466.6 ±2053.6 |
| Reagent 1 (Pre-dose) | 330.6 ±82.4 | 633.9 ± 181.5 | 977.1 ±449.0 | 1485.8 ±678.4 | 2269.1 ± 1175.8 | 3467.3 ±1763.1 |
| Reagent 1 (Same day dose) | 256.8 ± 98.9 | 52.4 ± 155.1 | 577.0 ± 256.5 | 777.6±501.7 | 1339.3 ±1115.6 | 1798.3 ±1528.6 |
| Reagent 2 (Pre-dose) | 267.9 ±76.4 | 502.0 ±138.9 | 730.6 ± 1803 | 912.3±365.5 | 1666.8 ±1263.2 | 2319.3 ±1682.9 |
| Reagent 2 (Same day dose) | 235.0 ±97.4 | 345.6 ±135.8 | 537.0 ±182.5 | 566.5±249.7 | 637.1 ±248.0 | 868.5 ±561.4 |
| Reagent 3 (Pre-dose) | 295.7 ±82.6 | 374.0 ±165.6 | 531.0 ±226.2 | 502.8±193.9 | 517.6 ±246.8 | 613.7 ±310.7 |
| Reagent 3 (Same day dose) | 307.4 ±92.9 | 517.9 191.6 | 1001.9 ±645.9 | 1336.2 ± 1037.1 | 2402.0 ±2359.5 | 337.6 ±3322.0 |
| Reagent 3 (After dose) | 256.5 ± 69.9 | 426.3 ±120.4 | 749.9 ±223.1 | 764.1 ±254.3 | 1086.6 ±525.9 | 1735.9 ±507.4 |
| Reagent 4 (Pre-dose) | 247.3 ±101.7 | 329.5 ±118.6 | 481.4 ±982 | 547.3 ±185.9 | 699.2 ±254.0 | 744.2 ±322.6 |
| Reagent 4 (Same day dose) | 242.4 ±67.3 | 354.0 ±104.0 | 562. ±160.3 | 562.0 ±160.3 | 713.0 ±3171.8 | 1274.7 ±702.7 |
| Reagent 4 (After dose) | 251.3 ±60.3 | 278.7 ±169.4 | 392.6 ±200.3 | 505.5 ±296.3 | 692.5 ±452.0 | 757.1 ±535.3 |

| | | | | | | |
|---|---|---|---|---|---|---|
| Distilled water: Control Krestin (PSK): Ag0 Reagent 2: Ag40 Reagent 3: Ag70 Reagent 4: Ag 150 Estimated weight (Average value ± Standard deviation) mg (n = 6) | | | | | | |

### 2) Experiment 2:[Function to Meth-A]

In this experiment, 5-week age female mice of MALB/c type (obtained from Japan SLC) were used as sample mice. The mice of MALB/c type were grafted with Meth-A tumor in its abodominal skin and dosed with the reagents , distilled water and PSK by free ingestion for twenty days from the grafted day. The mice were dosed with PSK by forced ingestion by using a probe. The ingestion dose was made on the same day from the grafted day to 24^{th} day (Same day dose), in one week from the grafted day to 24^{th} day (pre-dose), and in one week after the grafted day to 24^{th} day (after dose). For this period of time, the size (long and short radiuses) of tumor was periodically measured from the exterior, and the weight of the mice was measured. After twenty four days, the mice were killed by exsanguination, and the weight of tumor extracted from the mice was measured. The mice were provided for the experiment after preliminarily fed for one week after obtained in the same manner as the mice of ddY type.

### 3) Evaluation

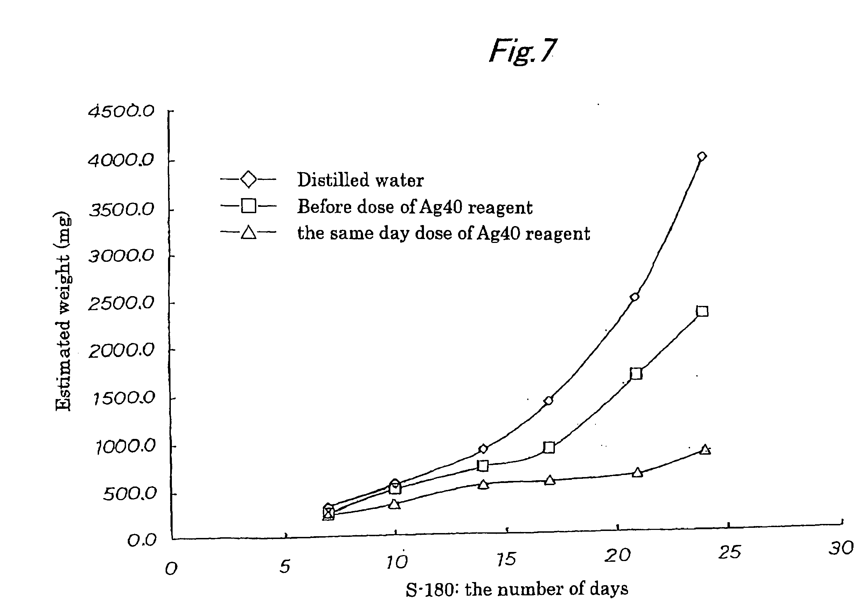

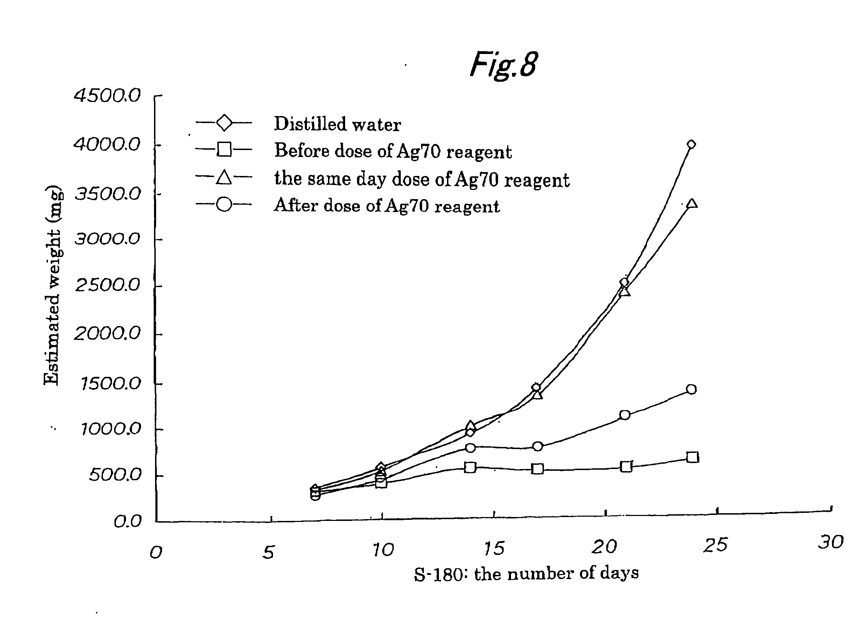

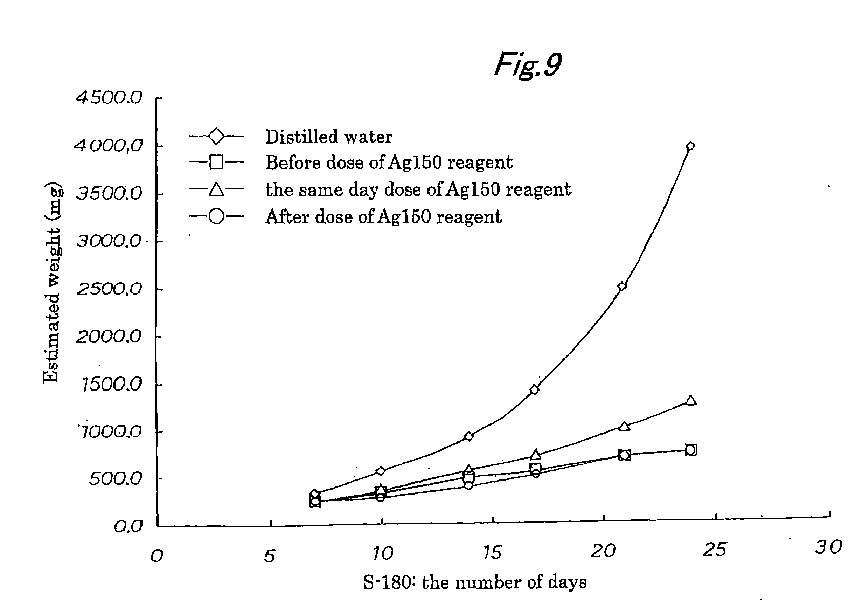

With respect to the suppressive effect of proliferation of S-180 tumor, the S-180 tumor in the distilled water group was increased in lapse of time, and the proliferation of S-180 tumor in the pre-dose group of PSK and the same day group ofAg0 reagent was slightly suppressed. (see Fig. 6) The proliferation of the tumor in the pre-dose group of Ag40 reagent and in the same day dose group of Ag40 reagent was significantly suppressed from ten days after grafted.(see Fig.7) The proliferation of the tumor in the pre-dose group of Ag70 reagent and in the after dose group ofAg70 reagent was significantly suppressed from seventeen days after grafted.(see Fig.8) Provided that, the proliferation of the tumor in the same day dose group of Ag70 was not partly suppressed. In an average value in the unpaired t-inspection, any significant difference was not found. The proliferation of the tumor in the respective groups of Ag150 reagent was significantly suppressed from ten days after grfated. (see Fig. 9)

In the table 5, there are shown the estimated weight and the real weight of S-180 tumor after dose of the reagents described above. The result represents the suppressive effect of proliferation of the S-180 tumor. In observation of a drink amount of water and a variation of weight of the grafted mice, any significant difference was not found in the mice dosed with the reagents Ag0, AG40, Ag70 and Ag150. Provided that, it has been found that the mice dosed with the reagents drank a large amount of water when compared with the mice dosed with distilled water. In addition, an increase of the weight of the mice dosed with the reagents was found, while a decrease of the weight of the mice caused by toxicity was not found.

With respect to the suppressive effect of proliferation of the Meth-A tumor, it has been found that the Meth-A tumor in the dose group of distilled water (the group of control) and in the dose group of PSK increased in lapse of time without any significant difference therein. It has been also found that the Meth-A tumor in each dose group of the reagents Ag0, Ag40, Ag70 and Ag150 reagent increased in lapse of time without any significant difference therein.

### Embodiment 3:

With respect to electrolyzed alkaline water containing the silver component of different concentration produced in the production apparatus of immunopotentiator, the distilled water (control) and PSK (positive control), the following experiment was carried out to confirm the function thereof to T-lymphocyte.

In this experiment, spleen extracted from mice dosed with the reagents was crushed by a slide-glass to prepare suspension of cells.(lympholite-M). The suspension of cells (Iympholite-M) was centrifugally separated by 200 rpm for twenty minutes at 20°C to obtain a group of T-lymphocyte cells. The T-lymphocyte cells were suspended in MEM medium to prepare 1 x 10⁶ cells/mL/well, added with (S-180) cell membrane of 50 µ L as antigen and incubated for twenty four hours at 37°C under the presence of 5 % CO₂. After the incubation, FITC marker CD 3e antibody and PE maker CD233 antibody and were reacted with the T-lymphocyte cells, and the activation of the T-lymphocyte cells was measured by FACS (flow cytometry) after washing.

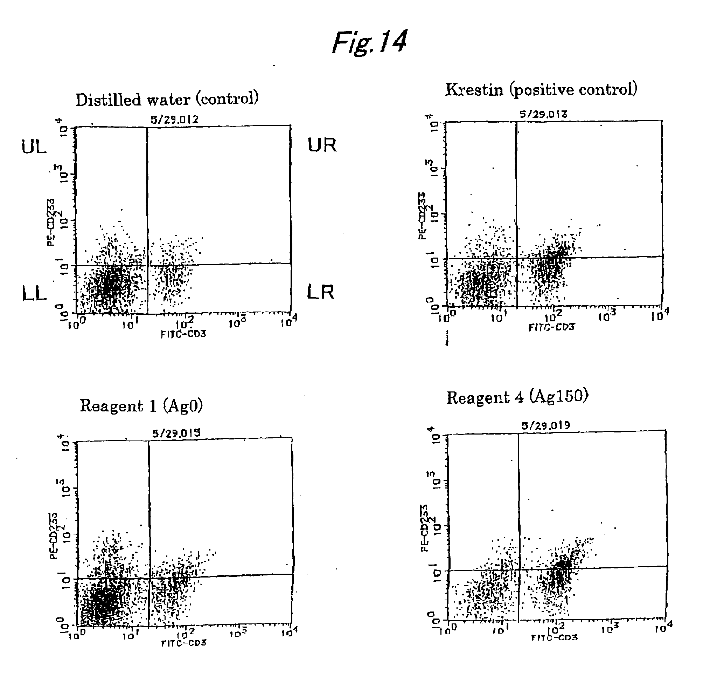

With respect to manifestation of CD3e indicative of cell cytopathy and CD233 indicative of activation of NK (natural killer), an individual representative of a typical example of the proliferation of tumor in the dosed group of each reagent was extracted from the result of the embodiment 2, and the CD manifestation of the spleen T-lymphocyte was analyzed by FACS. The activation function to the lymphocyte is shown in the following table 6, and a result of the analysis is shown in Fig. 14.

**Table 6**

| Activation function to lymphocyte: | | | | | | |
|---|---|---|---|---|---|---|
| Dosed reagent | Tumor (mg) | Spleen (mg) | Classification of activated lymphocyte (%) | | | |
| | | | UL | UR | LL | LR |
| Distilled water | 3102 | 788 | 8.86 | 4.39 | 72.77 | 13.98 |
| Krestin (before) | 94 | 231 | 4.99 | 13.15 | 47.55 | 34.31 |
| Reagent (before) | 660 | 458 | 11.07 | 5.09 | 69.23 | 14.61 |
| Reagent (before) | 18 | 244 | 5.21 | 2323 | 31.82 | 39.64 |

| | | | | | | |
|---|---|---|---|---|---|---|
| UL: Activated natural killer cell CD233(+) UR: Activated natural killer cell. CD233(+) and Activated cytotoxic T-cellCD3e(+) LL: Inactivated T-lymphocyte LR: Activated cytotoxic T-cell CD 3e(+) Distilled water. Control Krestin (PSK): Positive control Reagent 1: Ag0 reagent (electrolyzed alkaline water of pH 9.5 containing the silver component 0 ppb Reagent 4: Ag150 reagent (electrolyzed alkaline water of pH 9.5 containing the silver component 150 ppb | | | | | | |

In Fig. 14, the UR zone represents the activated natural killer cell CD233 (+) and the activated cytotoxic T-cell CD3e(+), the UL zone represents the activated natural killer cell C 233(+), the LR zone represents the activated cytotoxic T-cell CD3e(+), and the LL zone represents the inactivated lymphocyte. In the result of analysis by FACS, the inactivated T-lymphocyte of CD3e (-) and CD233(-) occupied 65 ~ 75 % of the control group and the dose group of Ag0 reagent respectively in which any suppression of the proliferation of the tumor was not found, and the activated T-lymphocyte of CD233(+) and CD3e(+) occupied 12 ~ 23 % of the dose group of Krestin (PSK: Positive control) and the dose group of Ag150 reagent respectively in which suppression of the proliferation of the tumor was found

The foregoing result represents the fact that a cellular immunity is activated by dose of the Ag150 reagent. The increase of the T-lymphocyte of the CD233 (+) indicative of activation of natural killer and the CD3e(+) indicative of activation of the cytotoxic T-cell suggests the fact that the cellular immunity function and the anti-tumor function are enhanced by the Ag40 ~ Ag150 reagents (electrolyzed alkaline water containing the silver component).

### Embodiment 4:

In this embodiment, electrolyzed alkaline and acidic water respectively containing a silver component of different concentration produced by an electrolytic cell with a silver electrode and an electrolytic cell with a partition membrane were used as a group A of reagent and a group B of reagent, electrolyzed alkaline water containing a silver component of different concentration produced by an electrolytic cell with a partition membrane and an electrolytic cell with a silver electrode was used as a group C of reagent, electrolyzed water produced by an electrolytic cell with a silver electrode was used as a group D of reagent, and silver solution prepared by sufficient stir of water added with silver powder was used as a group E of reagent. The following experiment (in vivo) was carried out to confirm the anti-tumor function of the foregoing reagents to the mouse grafted tumor (S-180).

### 1) Experiment:

In this experiment, 5-week age female mice of ddY type (obtained from Japan SLC) were used as sample mice. The mice of ddY type were grafted with S-180 cells of 1 x 10⁶ in its abodominal skin and doses with the reagents by free ingestion for fourteen days from the grafted day. During ingestion dose, the size (long and short radiuses) of tumor was periodically measured from the exterior, and the weight of the mice was measured. After fourteen days, the mice were killed by exsanguinations, and the weight of tumor extracted from the mice was measured.

Listed in the following table 7 is the group A of reagents (A1, A2), the group B of reagents (B1, B2, B3), the group C of reagents (C1, C2), the group d of reagents (D1, D2), and the group E of reagents (El, E2) were used for this experiment. In this experiment, distilled water was used as a group of controls. The mice were provided for the experiment after preliminarily fed for one week. For feeding, five mice were entered into a cage (32 x 231 x 13 cm) of polycarbonate and fed at a room temperature of 23 + 0.5°C under an ambience of 55 ± 5 % humidity. The cage was lightened for twelve hours from 8 a.m. to 8 p.m. and conditioned for free ingestion of water and feed.

**Table 7**

| Reagent | Kind (Breeding method) | pH | Ag(ppb) |
|---|---|---|---|
| A1 | AgEac(x-o) | 6 | 100 |
| A2 | AgEac(x-o) | 6 | 150 |
| B1 | AgEal(x-o) | 9 | 100 |
| B2 | AgEal(x-o) | 9 | 150 |
| B3 | AgEal(x-o) | 9 | 500 |
| C1 | AgEal(o-x) | 9 | 100 |
| C2 | AgEal(o-x) | 9 | 150 |
| D1 | Agion(x) | 7 | 100 |
| D2 | Agion(x) | 7 | 150 |
| E1 | Agaq | 7 | 100 |
| E2 | Agaq | 7 | 150 |

| | | | |
|---|---|---|---|
| AgEac (x - o): the group A of reagent AgEal (x - o): the group B of reagent AgEal (o-x): the group C of reagent Agion (x): the group D of reagent Agaq: the group E of reagent | | | |

### 2) Result:

To confirm the proliferation degree of tumor , the size (long and short radiuses) of tumor was periodically measured by a slide calipers, and the estimated weight of tumor was calculated by a formula, the long radius x the short radius (mm)²/2. The statistic processing was made by an unpaired t-inspection. The size of tumor extracted after dose of the reagents was measured by direct measurement of the weight of extracted tumor. These results are shown in the following tables 8, 9, 10, 11 and 12, respectively with respect to the groups A, B, C, D and E of reagents.

**Table 8**

| Suppressive effect of proliferation of grafted S-180 tumor (the group A) | | | | | | |
|---|---|---|---|---|---|---|
| | Reagent | Dose method | Estimated weight (mg) (Standard deviation) | | | Real weight (mg) |
| | | | 7th Day | 10th Day | 14th Day | 14th Day |
| 1 | Distilled Water | Simultaneous dose (non-grafted) | 0.00 (0.00) | 0.00 (0.00) | 0.00 (0.00) | 0.00 (0.00) |
| 2 | Distilled water | Simultaneous dose (grafted) | 304.71 (106.32) | 663.05 (302.06) | 1155.4 (343.37) | 1200.00 (610.00) |
| 3 | A2 | pre-dose (non-grafted) | 0.00 (0.00) | 0.00 (0.00) | 0.00 (0.00) | 0.00 (0.00) |
| 4 | A1 | pre-dose (grafted) | 161.78 (145.46) | 414.96 (256.65) | 652.53 (409.75) | 580.00 (380.00) |
| 5 | A2 | pre-dose (grafted) | 84.46 (38.90) | 337.57 (209.02) | 457.38 (301.25) | 580.00 (380.00) |
| 6 | A1 | Simultaneous dose (grafted) | 149.37 (99.68) | 336.34 (194.01) | 574.06 (386.82) | 570.00 (370.00) |
| 7 | A2 | Simultaneous dose (grafted) | 128.44 (69.86) | 305.23 (115.29) | 535.64 (277.62) | 610 (330.00) |

| | | | | | | |
|---|---|---|---|---|---|---|
| Grafted: with graft of S-180 cell Non-grafted: without graft of S-180 cell Deviation value: n = 8 | | | | | | |

**Table 9**

| Suppressive effect of proliferation of grafted S-180 tumor (the group B) | | | | | | |
|---|---|---|---|---|---|---|
| | Reagent | Dose method | Estimated weight (mg) (Standard deviation) | | | Real weight (mg) |
| | | | 7th Day | 10th Day | 14th Day | 14th Day |
| 8 | B2 | Pre-dose (non-grafted) | 0.00 (0.00) | 0.00 (0.00) | 0.00 (0.00) | 0.00 (0.00) |
| 9 | B1 | Pre-dose (grafted) | 169.18 (119.62) | 516.91 (326.82) | 788.66 (407.77) | 700.00 (570.00) |
| 10 | B2 | Pre-dose (grafted) | 168.46 (54.33) | 486.50 (240.44) | 692.21 (368.829 | 840.00 (430.00) |
| 11 | B3 | Pre-dose (grafted) | 118.07 (38.059 | 385.72 (145.35) | 571.51 (313.68) | 509.00 (430.00) |
| 12 | B1 | Simultaneous dose (grafted) | 173.47 (111.55) | 396.71 (289.91) | 602.49 (480.61 | 620.00 (420.00) |
| 13 | B2 | Simultaneous dose (grafted) | 183,7 (114.46) | 430.46 (281.49) | 750.95 (433.45) | 720.00 (430.00) |
| 14 | B3 | Simultaneous dose (grafted) | 147.72 (71.26) | 262.14 (136.19) | 638.01 (325.55) | 650.00 (320.00) |

| | | | | | | |
|---|---|---|---|---|---|---|
| Grafted: with graft of S-180 cell Non-grafted: without graft of S-180 cell Deviation value: n = 8 | | | | | | |

**Table 10**

| Suppressive effect of proliferation of grafted S-180 tumor (the group C) | | | | | | |
|---|---|---|---|---|---|---|
| | Reagent | Dose method | Estimated weight (mg) (Standard deviation) | | | Real weight (mg) |
| | | | 7th Day | 10th Day | 14th Day | 14th Day |
| 15 | C2 | pre-dose (non-grafted) | 0.00 (0.00) | 0.00 (0.00) | 0.00 (0.00) | 0.00 (0.00) |
| 16 | C1 | pre-dose (grafted) | 297.22 (95.03) | 577.79 (230.40) | 1000.48 (309.41) | 1050.00 (390.00) |
| 17 | C2 | pre-dose (grafted) | 314.77 (199.95) | 555.28 (347.77) | 1076.30 (519.76) | 1209.00 (540.00) |
| 18 | C1 | Simultaneous dose (grafted) | 297.92 (121.42) | 671.23 (277.66) | 1114.56 (445.52) | 1080.00 (500.00) |
| 19 | C2 | Simultaneous dose (grafted) | 293.30 (144.14) | 707.81 (291.54) | 1018.39 (530.32) | 1070.00 (530.00) |

| | | | | | | |
|---|---|---|---|---|---|---|
| Grafted: with graft of S-180 cell Non-grafted: without graft of S-180 cell Deviation value: n = 8 | | | | | | |

**Table 11**

| Suppressive effect of proliferation of grafted S-180 tumor (the group D) | | | | | | |
|---|---|---|---|---|---|---|
| | Reagent | Dose method | Estimated weight (mg) (Standard deviation) | | | Real weight |
| | | | 7th Day | 10m Day | 14th Day | 14th Day |
| 20 | D2 | pre-dose (non-grafted) | 0.00 (0.00) | 0.00 (0.00) | 0.00 (0.00) | 0.00 (0.00) |
| 21 | D1 | pre-dose (grafted) | 270.93 (165.23) | 620.01 (234.05) | 974.05 (247.44) | 1060.00 (280.00) |
| 22 | D2 | pre-dose (grafted) | 320.92 (160.43) | 658.77 (327.54) | 936.30 (395.66) | 1050.00 (450.00) |
| 23 | D1 | Simultaneous dose (grafted) | 308.59 (160.43) | 742.06 (304.28) | 1141.56 (380.81) | 1270.00 (390.00) |
| 24 | D2 | Simultaneous dose | 320.52 (138.95) | 694.86 (193.05) | 1146.53 (299.56) | 1270.00 (280.00) |

| | | | | | | |
|---|---|---|---|---|---|---|
| Grafted: with graft of S-180 cell Non-grafted: without graft of S-180 cell Deviation value: n = 8 | | | | | | |

**Table 12**

| Suppressive effect of proliferation of grafted S-180 tumor (the group E) | | | | | | |
|---|---|---|---|---|---|---|
| | Reagent | Dose method | Estimated weight (mg) (Standard deviation) | | | Real weight (mg) |
| | | | 7th Day | 10th Day | 14th Day | 14th Day |
| 25 | E2 | pre-dose (non-grafted) | 0.00 (0.00) | 0.00 (0.00) | 0.00 (0.00) | 0.00 (0.00) |
| 26 | E1 | pre-dose (grafted) | 281.72 (166.41) | 615.46 (325.24) | 1177.52 (455.89) | 1230.00 (570.00) |
| 27 | E2 | pre-dose (grafted) | 329.08 (187.76) | 712.71 (393.79) | 1131.41 (506.36) | 1270.00 (560.00) |
| 28 | E1 | Simultaneous dose (grafted) | 333.67 (257,97) | 653.50 (449.45) | 1255.86 (616.08) | 1130.00 (650.00) |
| 29 | E2 | Simultaneous dose (grafted) | 316.05 (175-42) | 678.72 (314.80) | 1098.38 (513.89) | 1110.00 (460.00) |

| | | | | | | |
|---|---|---|---|---|---|---|
| Grafted: with graft of S-180 cell Non-grafted: without graft of S-180 cell Deviation value: n = 8 | | | | | | |

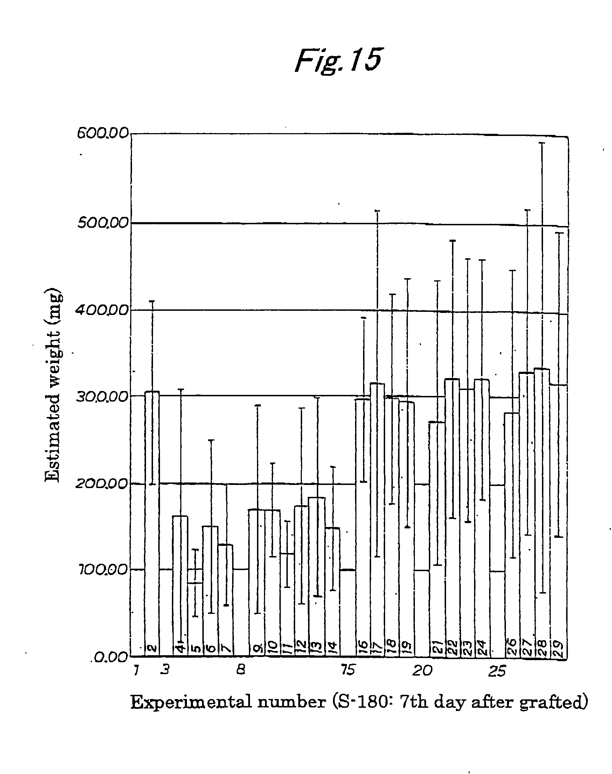

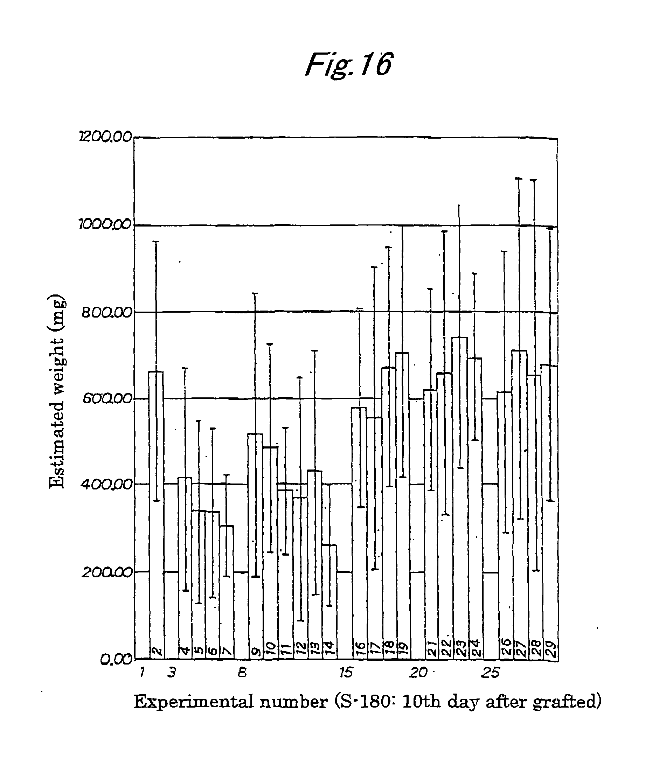

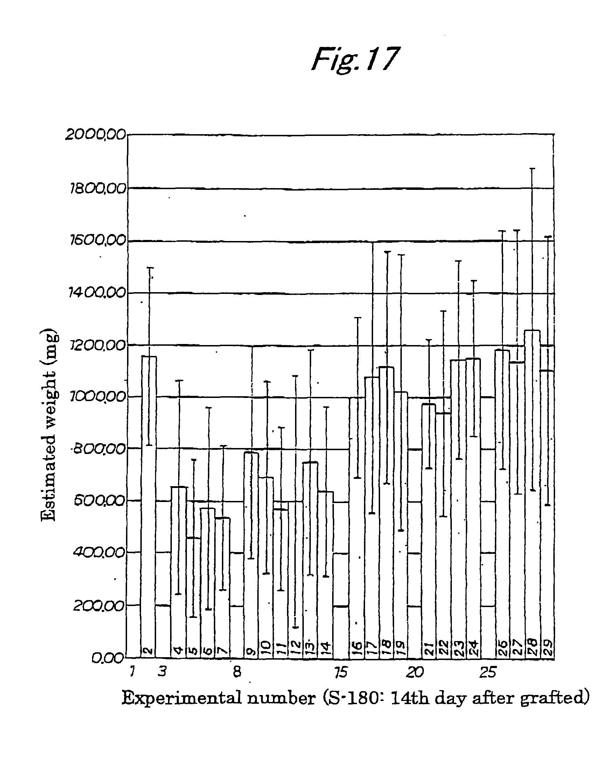

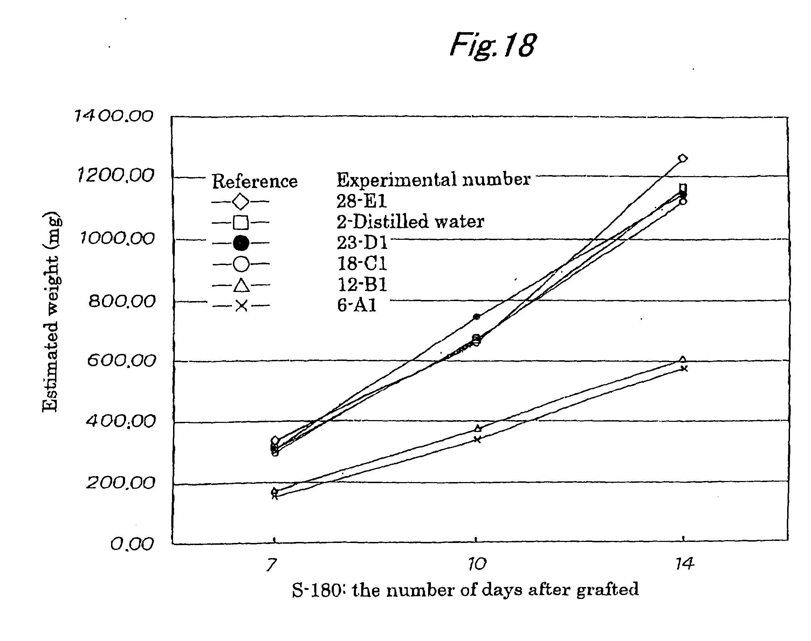

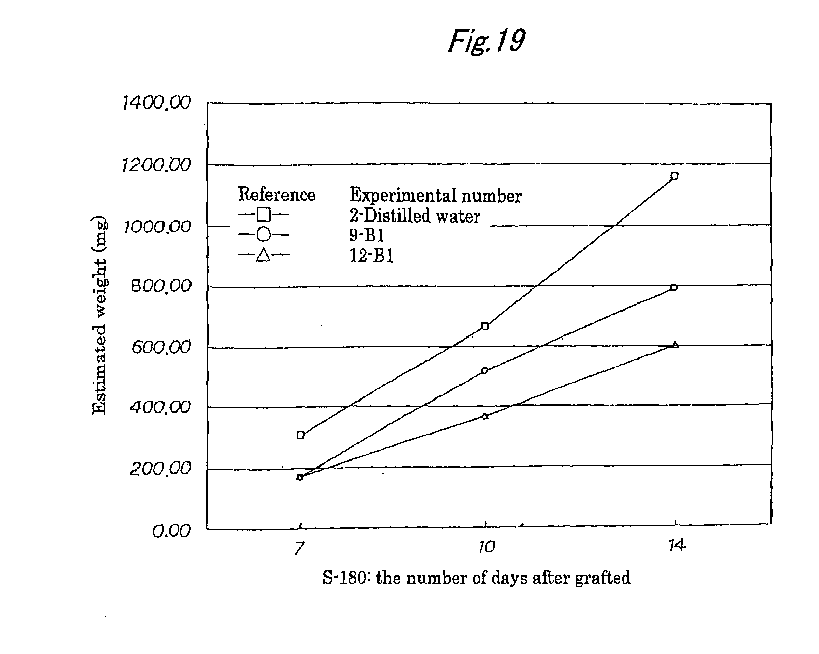

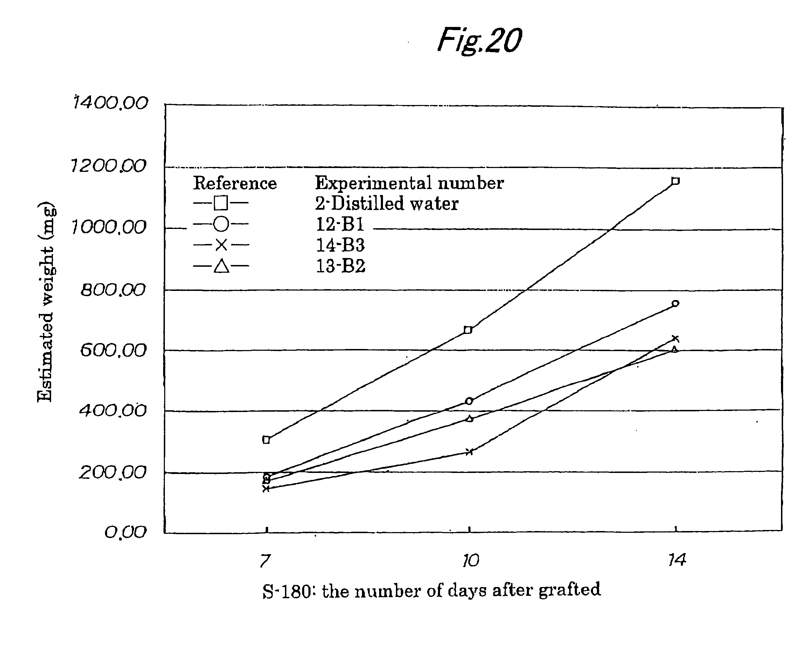

The results listed in the above tables are shown by graphs in Fig. 15 (7^{th} day), Fig. 16 (10^{th} day) and Fig.17 (14^{th} day), respectively. The result by difference of the production method of the reagents is shown in Fig. 18, the result by difference of dose times of the reagents is shown in Fig. 19, and the result by difference of the concentration of silver ion in the reagents is shown in Fig. 20.

### 3) Evaluation:

With respect to the difference of the production method of the reagents, a high suppressive effect of the proliferation of S-180 tumor was found in the groups A and B of the reagents. In the groups C, D and E of the reagents, however, any suppressive effect of the proliferation of S-180 tumor was not found. With respect to the difference of dose times, it has been found that the suppressive effect of the proliferation of S-180 tumor caused by simultaneous dose of the reagents is higher than that caused by pre-dose of the reagents. With respect to the difference of concentration of the silver ion in the reagents, the high suppressive effect of the proliferation of S-180 tumor has been found in the case that the concentration of the silver ion is 100 ppb or 500 ppb.

## Claims

1. An immunopotentiator to be used for enhancing an immune function of suppressing the proliferation of allogeneic tumor, the immunopotentiator containing, as an active ingredient, electrolyzed weakly alkaline water produced by electrolysis of water containing a silver component

2. An immunopotentiator as claimed in claim 1, wherein the electrolyzed weakly alkaline water is prepared at pH 9 as a standard value and contains the silver component of 100 ppb as a standard value.

3. An immunopotentiator as claimed in claim 1 or 2, wherein the electrolyzed weakly alkaline water is prepared at an oxide reduction potential of + 200 mV as a standard value.

4. An immunopotentiator to be used for enhancing an immune function of suppressing the proliferation of allogeneic tumor, the immunopotentiator containing, as an active ingredient, electrolyzed weakly acidic water produced by electrolysis of water containing a silver component.

5. An immunopotentiator as claimed in claim 4, wherein the electrolyzed weakly acidic water is prepared at pH 6 as a standard value and contains the silver component of 100 ppb as a standard value.

6. An immunopotentiator as claimed in claim 4 or 5, wherein the electrolyzed weakly acidic water is prepared at an oxide reduction potential of + 400 mV as a standard value.

7. A method of producing the immunopotentiator claimed in either one of claims 1 ~ 3, wherein the water containing the silver component is electrolyzed in a cathode chamber of an electrolytic cell with a partition membrane.

8. A method of producing the immunopotentiator claimed in either one of claims 1 ~ 3, wherein the water containing the silver component is electrolyzed in a cathode chamber of an electrolytic cell without any partition membrane and extracted therefrom.

9. A method of producing the immunopotentiator claimed in either one of claims 4 ~ 6, wherein the water containing the silver component is electrolyzed in an anode chamber of an electrolytic cell with a partition membrane.

10. A method of producing the immunopotentiator claimed in either one of claims 4 ~ 6, wherein the water containing the silver component is electrolyzed in an anode chamber of an electrolytic cell without any partition membrane and extracted therefrom.

11. A method of producing the immunopotentiator claimed in either one of claims 7 ~ 10, wherein the water containing the silver component is produced by electrolysis of pure water in an electrolytic cell provided therein with a silver electrode without any partition membrane.

12. A method of producing the immunopotentiator claimed in either one of claims 7 ~ 9, wherein the water containing the silver component is solution of silver powder solved in pure water.

13. An apparatus for carrying out the production method of the immunopotentiator claimed in claim 7 or 9, comprising a water treatment system composed of a water softener for softening general water such as tap water or natural water and a water purifier for purifying the softened water, a first electrolytic cell provided at its anode side with a silver electrode for electrolyzing the purified water, and a second electrolytic cell with a partition membrane for electrolyzing the electrolyzed water produced in the first electrolytic cell.

14. An apparatus for carrying out the production method of the immunopotentiator claimed in claim 8 or 10, comprising a water treatment system composed of a water softener for softening general water such as tap water or natural water and a water purifier for purifying the softened water, a first electrolytic cell provided at its anode side with a silver electrode for electrolyzing the purified water, and a second electrolytic cell for electrolyzing the electrolyzed water produced in the first electrolytic cell, the second electrolytic cell having anode and cathode chambers in connection with discharge conduits for discharging the electrolyzed water therefrom.
